# EUROPÄISCHE PATENTSCHRIFT

(11) **EP 2 654 630 B1**
(45) Veröffentlichungstag und Bekanntmachung des Hinweises auf die Patenterteilung: **07.04.2021**
(21) Anmeldenummer: 11794773.9
(22) Anmeldetag: 14.12.2011
(51) Int. Cl.: A61F 5/00

(54) **MEDIZINISCHE RESTRIKTIONSEINRICHTUNG FÜR HOHLORGANE EINES KÖRPERS**
MEDICAL RESTRICTION DEVICE FOR HOLLOW ORGANS OF A BODY
DISPOSITIF DE RESTRICTION MÉDICAL POUR ORGANES CREUX D'UN CORPS

(30) Priorität: 23.12.2010 EP 10196846
(43) Veröffentlichungstag der Anmeldung: 30.10.2013
(73) Patentinhaber: Q Medical International AG, 8260 Stein am Rhein (CH)
(72) Erfinder: SZEWCZYK, Tomasz, PL-91-162 Lodz (PL); CLAESSENS, Frank, B-3680 Maaseik (BE)
(74) Vertreter: dompatent von Kreisler Selting Werner - Partnerschaft von Patent- und Rechtsanwälten mbB
(86) Internationale Anmeldenummer: PCT/EP2011/072801
(87) Internationale Veröffentlichungsnummer: WO 2012/084653

(56) Entgegenhaltungen:
- EP-A2- 1 036 545
- EP-A2- 1 829 505
- WO-A1-2010/086335
- WO-A2-02/096326
- US-A- 5 269 803
- US-A1- 2009 306 463

## Beschreibung

Die Erfindung betrifft eine medizinische Restriktionseinrichtung für Hohlorgane eines Körpers gemäß Anspruch 1.

Derartige medizinische Restriktionseinrichtungen für Hohlorgane sind beispielsweise in der EP 1 778 098 A beschrieben. Die bekannte Restriktionseinrichtung weist ein längliches flexibles, aber nicht in Längsrichtung dehnbares Element mit einem ersten und einem zweiten Ende auf.

Eine Schließeinrichtung arretiert das längliche Element ringförmig in einer einzigen vorbestimmten Durchmesserposition. Für unterschiedliche Durchmesser müssen unterschiedliche Restriktionseinrichtungen vorgehalten werden. Eine abziehbare flexible Spitze an dem ersten Ende dient als Einführhilfe des länglichen Elementes zum Platzieren der Restriktionseinrichtung am Hohlorgan.

Die WO 2010 / 086335 offenbart eine implantierbare medizinische Vorrichtung, die ein flexibles Band umfasst, das dazu bestimmt ist, um ein Organ herum angeordnet zu werden, wobei die medizinische Vorrichtung ein Positionierungsmittel umfasst, das mit einem gekrümmten Versteifungselement mit einer Steifigkeit versehen ist, die größer ist als die des flexiblen Bandes, wobei das Positionierungsmittel dazu bestimmt ist, um das Organ herum eingeführt zu werden, um im Wesentlichen den Weg für die Anordnung des flexiblen Bandes um das Organ herum zu markieren. Ausgehend von einem solchen Stand der Technik liegt der Erfindung die Aufgabe zugrunde, eine verbesserte Restriktionseinrichtung zu schaffen, die bei der Applikation einfacher handhabbar ist.

Zur Lösung dieser Aufgabe dienen erfindungsgemäß die Merkmale des Anspruchs 1.

Die Erfindung hat folgende Vorteile:
Die Schließeinrichtung kann das längliche Element in mehreren wählbaren Durchmesserpositionen reversibel arretieren. Beispielsweise kann die Restriktionseinrichtung in vier Durchmesserposi-tionen arretiert werden.

Das längliche Element weist hierzu die Öffnung an dem zweiten Ende, die mit einer von mehreren, z.B. vier Einkerbungen, an dem ersten Ende zusammenwirken kann, um die Restriktionseinrichtung in einer von mehreren Rastpositionen zu verriegeln.

Hierzu sind die Einkerbungen und die Öffnung einander derart angepasst, dass die dadurch gebildete Schließeinrichtung eine Arretierung in den jeweiligen Positionen erlaubt. Dadurch ist die Restriktionseinrichtung universeller, nämlich für unterschiedliche Durchmesser verwendbar und ist an den Durchmesser des Hohlorgans anpassbar.

Weiterhin ist vorgesehen, dass die Schließeinrichtung an dem ersten Ende des länglichen Elementes der Öffnung am zweiten Ende des länglichen Elementes angepasste Einkerbungen aufweist, und dass die Öffnung am zweiten Ende mit den Einkerbungen elastisch zusammenwirkt und in den Einkerbungen einrastet, um das längliche Element ringförmig in einer von mehreren wählbaren Durchmesser-positionen zu arretieren.

Die Einkerbungen sind elastisch und lassen auch eine nachträgliche Erweiterung der Restriktionseinrichtung vor dem Abtrennen der Spitze zu, z.B. durch Arretieren der Schließeinrichtung in der in Richtung Spitze nächstfolgenden Einkerbung.

Aufgrund des flexiblen Materials ist es möglich, nach dem Schließen der Restriktionseinrichtung noch die Rastposition zu verändern und eine andere Rastposition einzunehmen, die durch die Einkerbungen in dem länglichen Element bestimmt sind.

Bei einer bevorzugten Ausführungsform ist vorgesehen, dass die Einkerbungen und die Öffnung der Schließeinrichtung am zweiten Ende des länglichen Elementes formschlüssig, z.B. konisch oder kegelförmig, aneinander angepasst sind. Auf diese Weise wird die Restriktionseinrichtung einerseits in den unterschiedlichen Durchmesserpositionen sicher durch die Schließeinrichtung gehalten, erlaubt aber dennoch mit einem erhöhten Kraftaufwand ein Überwinden der Rastpositionen zur Erweiterung des Durchmessers der Restriktionseinrichtung. Auf diese Weise ist die einmal angenommene Rastposition auch reversibel, wenn z.B. zunächst versehentlich eine zu enge Durchmesserposition eingestellt worden ist.

Die Restriktionseinrichtung kann auch laparoskopisch einsetzbar sein und durch eine Öffnung eines Trokars in den Körper einführbar sein. Hierzu sind die Dimensionen der Restriktionseinrichtung an den Nenndurchmesser eines Trokars, z.B. 10 mm Öffnungsdurchmesser angepasst, so dass die Restriktionseinrichtung im offenen Zustand durch den Trokar einführbar ist.

Aufgrund der Spitze mit einem Verstärkungskern kann die Restriktionseinrichtung besser um das Hohlorgan herumgeführt werden und kann einfacher das elastische und faserige Bindegewebe, das ein Hohlorgan umgibt und in Position hält, durchstoßen. In Verbindung mit der Öffnung an dem zweiten Ende des länglichen Elementes kann die Spitze einfacher durch die Öffnung hindurchgeführt werden, um die Restriktionseinrichtung in der Schließstellung der Schließeinrichtung in einer vorgegebenen oder wählbaren Position zu arretieren. In vorteilhafter Weise ist die Spitze zunächst einstückig mit dem ersten Ende verbunden und ist nach Arretierung des länglichen Elementes durch die Schließeinrichtung abtrennbar, so dass die Spitze nicht am Hohlorgan verbleiben muss und die Restriktionseinrichtung ringförmig das Hohlorgan umschließen kann. Die Spitze kann vorzugsweise an einer Einkerbung beispielsweise mit einer Schere abgetrennt werden, nachdem die Restriktionseinrichtung in ihrer Schließposition durch die Schließeinrichtung arretiert ist.

Vorzugsweise ist vorgesehen, dass das längliche Element aus einem in geringem Umfang dehnbaren Material besteht. Die dabei zulässige maximale Dehnung liegt zwischen ca. 1% und ca. 20%, vorzugsweise zwischen ca. 1% und 10%.

Bei einer weiteren Weiterbildung der Erfindung ist vorgesehen, das zweite Ende eine nach dem Schließen der Restriktionseinrichtung abtrennbare Zuglasche aufweist.

Die Zuglasche kann von einem geeigneten Greifinstrument gehalten werden und gibt einen ausreichenden Gegenhalt, wenn die Spitze des länglichen Elementes in die Öffnung am zweiten Ende eingeschoben wird und letztlich durch die Öffnung hindurch gezogen wird.

Nach dem Arretieren der Restriktionsvorrichtung kann auch die Zuglasche von der Restriktionseinrichtung abgetrennt werden. Hierzu kann eine Sollschnittstelle an dem den länglichen Element zugewandten Ende der Zuglasche vorgesehen sein.

An dem zweiten Ende können mindestens zwei Fixierösen angeordnet sein. Diese in der Nähe der Öffnung an dem zweiten Ende angeordneten Fixierösen erlauben es nach dem exakten Positionieren der Restriktionseinrichtung diese an dem Hohlorgan festzunähen.

Die Zuglasche kann an dem zweiten Ende eine reibungserhöhende Oberfläche aufweisen, damit das Greifinstrument die Zuglasche besser halten kann.

Die Restriktionseinrichtung ist radiopaque (strahlenundurchlässig). Dabei ist vorzugsweise die gesamte Restriktionseinrichtung radiopaque. Dies ist von Bedeutung, falls die abtrennbare Spitze verlorengeht, da sie dann im Körper lokalisiert werden kann.

Bei einem bevorzugten Ausführungsbeispiel ist vorgesehen, dass der Verstärkungskern oder die Spitze des Verstärkungskerns in Richtung der Krümmung der in Schließstellung ringförmigen Restriktionseinrichtung vorgekrümmt ist. Der starre Verstärkungskern kann auch aus Metall oder hartem Kunststoff bestehen und ist im Übrigen von dem Silikonmaterial der Restriktionseinrichtung umgeben. Die Krümmung der Spitze in Verbindung mit ihrer Steifheit aufgrund des Verstärkungskerns erleichtert die Einführung der Spitze in die Öffnung an dem zweiten Ende des länglichen Elementes und erleichtert das Arretieren der Schließeinrichtung.

Die Restriktionseinrichtung besteht vorzugsweise aus einem transparenten oder eingefärbten Material. Als Material wird bevorzugt ein medizinisches Silikon z.B. unrestricted NuSil-Slikon verwendet.

Die Restrektionseinrichtung kann zumindest im Bereich des länglichen Elementes eine durch Tempern bei einer vorgegebenen Temperatur vorgegebene Krümmung aufweisen.

Im Folgenden werden unter Bezugnahme auf die Zeichnungen Ausführungsbeispiele der Erfindung näher erläutert.

Es zeigen:
Fig. 1 : eine Seitenansicht auf ein erstes Ausführungsbeispiel einer medizinischen Restriktionseinrichtung,
Fig. 2 : zeigt eine Draufsicht auf das Ausführungsbeispiel der Fig. 1,
Fig. 3 : einen Schnitt entlang der Linie A-A in Fig. 2, und
Fig. 4 : eine perspektivische Ansicht.

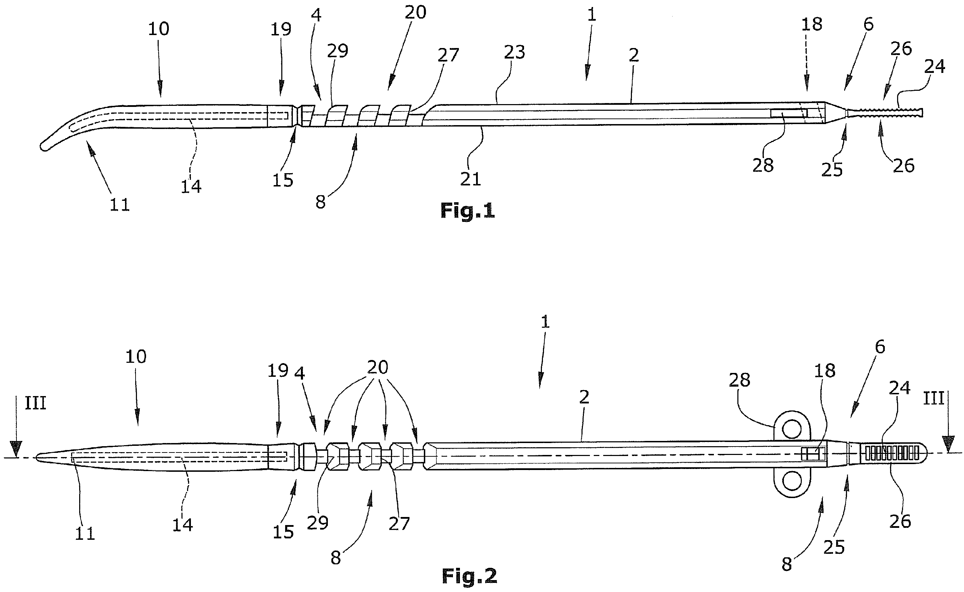

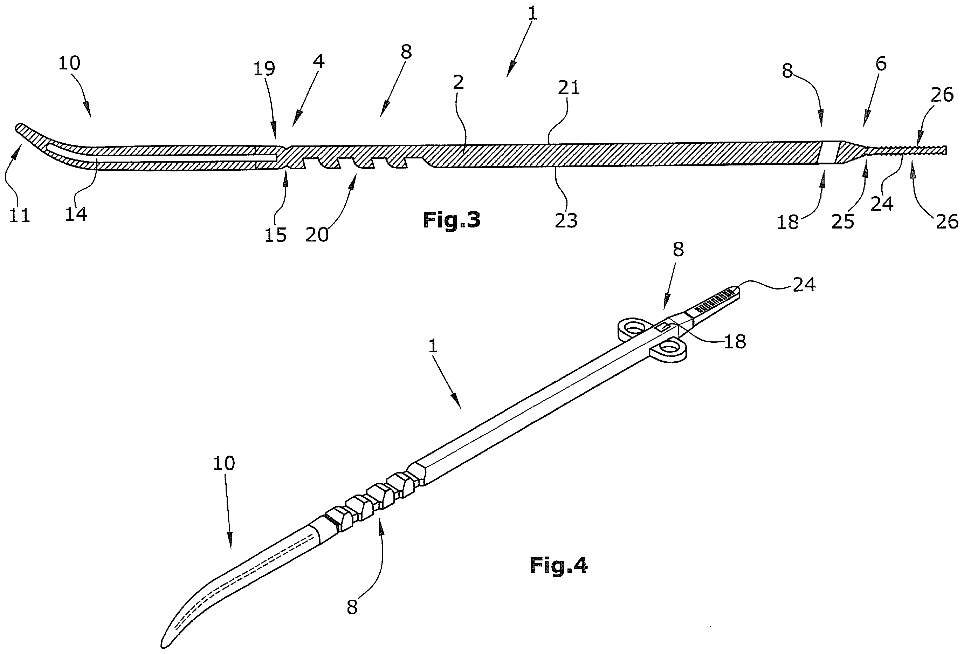

Die in den Fign. 1 bis 4 gezeigte medizinische Restriktionseinrichtung 1 für Hohlorgane eines Körpers weist ein längliches flexibles Element 2 mit einem ersten Ende 4
und einem zweiten Ende 6 auf. An dem ersten Ende 4 ist eine abtrennbare Spitze 10 fest mit dem länglichen Element 2 verbunden. Beispielsweise hat das längliche Element im Querschnitt eine Breite von ca. 4,5 mm und eine Höhe von ca. 3 mm.

Eine Schließeinrichtung 8 erlaubt, das erste Ende 4 mit dem zweiten Ende 6 in einer vorbestimmten wählbaren Durchmesserposition zu arretieren, wobei sich das längliche Element 2 dabei ringförmig um ein Hohlorgan erstreckt.

Die Spitze 10 kann nach dem Arretieren des länglichen Elementes 2 durch die Schließeinrichtung 8 an einer vorgesehenen Schnittstelle 15 abgetrennt werden. Die Spitze 10 kann an den dem länglichen Element 2 zugewandten Ende Riffelungen für ein Greifinstrument aufweisen, die bei der Handhabung der Restriktionseinrichtung 1 mit einem Greifinstrument reibungserhöhend wirken, so dass das Greifinstrument nicht abrutschen kann. In Richtung auf die Spitze 10 kann neben der Schnittstelle 15, wenn die Restriktionseinrichtung 1 nicht einstückig hergestellt wird, ein Kupplungselement 19 angeordnet sein, mit dessen Hilfe die Spitze 10 nachträglich, z.B. durch Verkleben mit dem ersten Ende 4 des länglichen Elementes 2 verbunden werden kann.

Die Spitze 10 hat einen Verstärkungskern 14 aus einem starren Material, beispielsweise Metall oder hartem Kunststoff.

Der Verstärkungskern 14 kann zunächst geradlinig verlaufen oder zum freien Ende 11 der Spitze 10 hin leicht in Richtung zur Innenseite 21 der Restriktionseinrichtung 1 gekrümmt sein. Das Verstärkungselement 14 ist von dem Material der Restriktionseinrichtung 1 umgeben, das vorzugsweise aus medizinischem Silikon (NuSil) besteht. Das Silikonmaterial läuft an dem distalen Ende der Spitze 10 spitz zu und geht über das Verstärkungselement 14 hinaus, so dass das freie Ende 11 der Spitze 10 flexibel beweglich ist.

Das Schließelement 8 besteht aus einer vorzugsweise in der Draufsicht gemäß Fig. 2 rechteckig gestalteten, schräg in dem länglichen Element 2 am zweiten Ende 6 angeordneten Öffnung 18, sowie mehreren Einkerbungen 20 in dem Material des länglichen Elementes 2. Die Öffnung 18 verläuft z.B. unter einem Winkel von 15° zur Innenseite 21 des länglichen Elementes 2. Die Einkerbungen 20 bilden bei dem Ausführungsbeispiel vier Rastpositionen, indem die Öffnung 18 in einer der Einkerbungen 20 einrastet. Hierzu wird die Spitze 10 durch die Öffnung 18 gezogen, bis die gewünschte Rastposition erreicht ist. Mit Hilfe der vier Einkerbungen 20 besteht die Möglichkeit, vier unterschiedliche Durchmesser der Restriktionseinrichtung 1 einzustellen. Die Restriktionseinrichtung 1 umschliesst dabei das Hohlorgan ringförmig. Die Einkerbungen 20 sind durch Aussparungen in dem länglichen Element 2 gebildet. Bei dem Ausführungsbeispiel der Fign. 1 bis 4 sind die Aussparungen in der Außenseite 23 des länglichen Elementes 2 vorgesehen, während die Innenseite 21 des länglichen Elementes 2 durchgängig in einer Ebene liegt. Zusätzlich können die Einkerbungen 20, wie aus Fig. 1 in der Draufsicht ersichtlich, durch seitliche Aussparungen im Material gebildet sein. Die Einkerbungen 20 sind in ihrer Form der Form der Öffnung 18 angepasst, so dass die Öffnung 18 formschlüssig in den Einkerbungen 20 einrasten und diese umschließen kann. Beispielsweise verlaufen, wie aus Fig. 2 ersichtlich, die Seitenwände 27 der Einkerbung 20 unter einem Winkel, der mit dem Winkel der schrägverlaufenden Öffnung 18, z.B. 15°, übereinstimmt.

Der gegenseitige Abstand der Einkerbung 20 kann beispielsweise 5 mm betragen. Die Länge des Elementes 2 zwischen der Öffnung 18 und der äusseren Einkerbung 20 beträgt beispielsweise zwischen 80 mm und 90 mm, vorzugsweise 83 mm. Die Gesamtlänge der Restriktionseinrichtung 1 beträgt beispielsweise 150 mm bis 160 mm, vorzugsweise 154 mm.

An dem ersten Ende 6 ist am länglichen Element 2 eine Zuglasche 24 angeformt, die eine abgeflachte Form aufweist und an der Ober- und Unterseite Riffelungen 26 aufweisen kann, um die Haftung eines Greifinstrumentes zu erhöhen. Die Zuglasche 26 ist einstückig mit dem länglichen Element 2, kann aber an einer Sollschnittstelle 25 nach dem VerSchließen der Restriktionseinrichtung 1 abgetrennt werden.

In der Nähe der Öffnung 18 kann eine Fixieröse 28 angeordnet sein, mit der die Restriktionseinrichtung 1 nach dem Anordnen um das Hohlorgan an diesem z.B. durch Nähen befestigt werden kann. Die Einkerbungen 20 sind auf der dem ersten Ende 4 zugewandten Seite an ihren Flanken abgerundet, damit das zweite Ende mit der Öffnung 18 einfacher in die engeren Arretierpositionen gezogen werden kann.

Dagegen sind die dem ersten Ende 4 zugewandten Flanken geradlinig, um eine sichere Arretierposition zu gewährleisten. Dennoch besteht die Möglichkeit, aufgrund der Flexibilität des Materials im Bedarfsfall eine engere Rastposition mit erhöhtem Kraftaufwand zu verlassen, um eine Arretierposition mit grösserem Durchmesser der Restriktionseinrichtung 1 einzustellen.

Die Restriktionseinrichtung 1 gemäss allen Ausführungsbeispielen weist im Querschnitt einen Durchmesser auf, der es erlaubt, die Restriktionseinrichtung 1 im geöffneten Zustand durch die Öffnung eines Trokars in den Körper einzuführen. Da die Fixierösen 28 aus flexiblem Silikonmaterial sind, können sich die Fixierösen 28 verbiegen und behindern dadurch nicht die Einführung der Restriktionseinrichtung 1 in einen Trokar.

Die Restriktionseinrichtung 1 ist radiopaque. Dies bedeutet, dass sie strahlungsundurchlässig ist und dadurch, z.B. bei einer Röntgenuntersuchung sichtbar ist.

Das Silikonmaterial der Restriktionseinrichtung 1 kann transparent oder eingefärbt sein. Beispielsweise kann die Restriktionseinrichtung 1 blau eingefärbt sein, um sich vom Körpergewebe bei Betrachtung durch das menschliche Auge oder durch eine Kamera auf einem Bildschirm besser unterscheidbar zu sein.

Die Restriktionseinrichtung 1 kann zumindest im Bereich des länglichen Elementes durch Tempern bei einer vorgegebenen Temperatur eine vorgegebene Krümmung erhalten.

## Patentansprüche

1. Medizinische Restriktionseinrichtung (1) für Hohlorgane eines Körpers, mit
- einem länglichen flexiblen Element (2) mit einem ersten (4) und einem zweiten Ende (6),
- einer Schließeinrichtung (8), die das längliche Element (2) ringförmig in einer vorbestimmten Durchmesserposition arretiert, und
- einer Spitze (10) als Einführhilfe an dem ersten Ende (4) des länglichen Elementes (2) zum Platzieren der Restriktionseinrichtung (1) am Hohlorgan, wobei
- eine Öffnung (18) am zweiten Ende (6) des länglichen Elementes (2) als Teil der Schließeinrichtung (8) vorgesehen ist,
- die Spitze (10) mit dem ersten Ende (4) des länglichen Elementes (2) in die Öffnung (18) am zweiten Ende (6) des länglichen Elementes (2) zum Arretieren der Schließeinrichtung (8) einschiebbar ist, und
- die Spitze (10) mit dem ersten Ende (4) fest verbunden ist und nach Arretierung des länglichen Elementes (2) durch die Schließeinrichtung (8) abtrennbar ist,
wobei
- die Spitze einen Verstärkungskern aus steifem Material aufweist und wobei die Schließeinrichtung (8) an dem ersten Ende (4) des länglichen Elementes (2) der Öffnung (18) am zweiten Ende (6) des länglichen Elementes (2) angepasste Einkerbungen aufweist, dass die Öffnung (18) am zweiten Ende (6) mit den Einkerbungen (20) elastisch zusammenwirkt und in den Einkerbungen (20) einrastet, um das längliche Element (2) ringförmig in einer von mehreren wählbaren Durchmesserpositionen zu arretieren, wobei die Einkerbungen (20) elastisch sind und auch eine nachträgliche Erweiterung des Durchmessers der Restriktionseinrichtung (1) vor dem Abtrennen der Spitze (10) zulassen und wobei die Öffnung (18) in dem länglichen Element (2) vorgesehen ist, wobei das längliche Element aus einem dehnbaren Material besteht, wobei die maximale Dehnung 1% bis 20% beträgt.

2. Vorrichtung nach Anspruch 1, wobei das zweite Ende (6) eine nach dem Schließen der Restriktionseinrichtung (1) abtrennbare Zuglasche (24) aufweist.

3. Vorrichtung nach Anspruch 2, wobei die Zuglasche (24) an dem zweiten Ende (6) eine reibungserhöhende Oberfläche (26) aufweist.

4. Vorrichtung nach einem der Ansprüche 1-3, wobei das längliche Element (2) aus einem in geringem Umfang dehnbaren Vollmaterial besteht.

5. Vorrichtung nach einem der Ansprüche 1-4, wobei die Restriktionseinrichtung (1) laparoskopisch einsetzbar ist und durch die Öffnung eines Trokars in den Körper einführbar ist.

6. Vorrichtung nach einem der Ansprüche 1-5, wobei an dem zweiten Ende mindestens zwei Fixierösen (28) angeordnet sind.

7. Vorrichtung nach einem der Ansprüche 1-6, wobei die Restriktionseinrichtung (1) radiopaque ist.

8. Vorrichtung nach einem der Ansprüche 1-7, wobei der Verstärkungskern (14) oder die Spitze (16) des Verstärkungskerns (14) in Richtung der Krümmung der in Schließstellung ringförmigen Restriktionseinrichtung (1) vorgekrümmt ist, und dass der Verstärkungskern (14) Metall oder harten Kunststoff aufweist.

9. Vorrichtung nach einem der Ansprüche 1-8, wobei die Einkerbungen (20) und die Öffnung (18) der Schließeinrichtung (8) formschlüssig aneinander angepasst sind.

10. Vorrichtung nach einem der Ansprüche 1-9, wobei die Restriktionseinrichtung (1) aus einem transparenten oder eingefärbten Material besteht.

11. Vorrichtung nach einem der Ansprüche 1-10, wobei zumindest das längliche Element (2) der Restriktionseinrichtung (1) durch Tempern bei einer vorgegebenen Temperatur eine vorgegebene Krümmung aufweist.

## Claims

1. Medical restriction device (1) for hollow organs of a body, comprising
- a longitudinal flexible element (2) having a first (4) and a second end (6),
- a closure device (8) for locking the longitudinal element (2) annularly in a predetermined diameter position, and
- a tip (10) as an inlet guide at the first end (4) of the longitudinal element (2) for positioning the restriction device (1) at the hollow organ,
- an opening (18) provided at the second end (6) of the longitudinal element (2) as a part of the closure device (8),
- the tip (10) is adapted to be inserted together with the first end (4) of the longitudinal element (2) into the opening (18) at the second end (6) of the longitudinal element (2) in order to lock the closure device (8), and
- the tip (10) is firmly connected with the first end (4) and can be severed after the longitudinal element (2) has been locked by the closure device (6),
wherein
- the tip (10) comprises a reinforcing core (14) of rigid material, and the closure device (8) at the first end (4) of the longitudinal element (2) has notches adapted to the opening (18) at the second end (6) of the longitudinal element (2), wherein the opening (18) at the second end (6) elastically cooperates with the notches (20) and engages the notches (20) in order to lock the longitudinal element (2) annularly in one of a plurality of selectable diameter positions, wherein the notches (20) are elastic and also enable a posterior widening of the diameter of the restriction device (1) prior to severing the tip (10), and wherein the opening (18) is provided in the longitudinal element (2), wherein the longitudinal element is made of an extensible material, wherein the maximum extension is 1% to 20%.

2. Device of claim 1, wherein the second end comprises a pull tab (24) adapted to be severed after the restriction device (1) is closed.

3. Device of claim 2, wherein the pull tab (24) at the second end (6) has a surface (26) that increases friction.

4. Device of one of claims 1 - 3, wherein the longitudinal element (2) is made of a solid material that is extensible to a low degree.

5. Device of one of claims 1 - 4, wherein the restriction device (1) is adapted for laparoscopic use and is adapted for insertion into the body via the opening of a trocar.

6. Device of claim 1 - 5, wherein at least two fixation eyelets (28) are provided at the second end.

7. Device of claim 1 - 6, wherein the restriction device (1) is radio-opaque.

8. Device of one of claims 1 - 7, wherein the reinforcing core (14) or the tip (16) of the reinforcing core (14) is pre-curved towards the curvature of the restriction device (1) which is annular in the closed position, and wherein the reinforcing core (14) includes metal or hard plastics.

9. Device of one of claims 1 - 8, wherein the notches (20) and the opening (18) of the closure device (8) are matched in a form fitting manner.

10. Device of one of claims 1 - 9, wherein the restriction device (1) is made from a transparent or colored material.

11. Device of one of claims 1 - 10, wherein at least the longitudinal element (2) of the restriction device (1) has a predetermined curvature caused by tempering at a predetermined temperature.

## Revendications

1. Dispositif de restriction médical (1) pour organes creux d'un corps, doté
- d'un élément flexible allongé (2) doté d'une première (4) et d'une deuxième extrémité (6),
- d'un dispositif de fermeture (8), lequel verrouille l'élément allongé (2) en forme annulaire dans une position diamétrale prédéterminée, et
- d'une pointe (10) en tant qu'aide d'insertion à la première extrémité (4) de l'élément allongé (2) afin de placer le dispositif de restriction (1) sur l'organe creux, dans lequel
- une ouverture (18) est prévue à la deuxième extrémité (6) de l'élément allongé (2) comme partie du dispositif de fermeture (8)
- la pointe (10) peut coulisser avec la première extrémité (4) de l'élément allongé (2) dans l'ouverture (18) à la deuxième extrémité (6) de l'élément allongé (2) afin de verrouiller le dispositif de fermeture (8), et
- la pointe (10) est reliée fixement à la première extrémité (4) et est détachable après verrouillage de l'élément allongé (2) par le dispositif de fermeture (8),
dans lequel
- la pointe comporte un noyau de renforcement en matériau rigide et dans lequel le dispositif de fermeture (8) comporte à la première extrémité (4) de l'élément allongé (2) des échancrures adaptées à l'ouverture (18) à la deuxième extrémité (6) de l'élément allongé (2), l'ouverture (18) à la deuxième extrémité (6) coopère élastiquement avec les échancrures (20) et s'emboîte dans les échancrures (20) afin de verrouiller l'élément allongé (2) en forme annulaire dans une de plusieurs positions diamétrales sélectionnables, dans lequel les échancrures (20) sont élastiques et permettent également un élargissement postérieur du diamètre du dispositif de restriction (1) avant de détacher la pointe (10) et dans lequel l'ouverture (18) est prévue dans l'élément allongé (2), dans lequel l'élément allongé est constitué d'un matériau étirable, dans lequel l'étirement maximal vaut entre 1 % et 20 %.

2. Appareil selon la revendication 1, dans lequel la deuxième extrémité (6) comporte une patte de traction (24) détachable après la fermeture du dispositif de restriction (1).

3. Appareil selon la revendication 2, dans lequel la patte de traction (24) comporte à la deuxième extrémité (6) une surface (26) augmentant la friction.

4. Appareil selon l'une des revendications 1-3, dans lequel l'élément allongé (2) est constitué d'un matériau plein étirable dans une moindre mesure.

5. Appareil selon l'une des revendications 1-4, dans lequel le dispositif de restriction (1) est utilisable de façon laparoscopique et est insérable dans le corps à travers l'ouverture d'un trocart.

6. Appareil selon l'une des revendications 1-5, dans lequel au moins deux œillets de fixation (28) sont disposés à la deuxième extrémité (6).

7. Appareil selon l'une des revendications 1-6, dans lequel le dispositif de restriction (1) est radiopaque.

8. Appareil selon l'une des revendications 1-7, dans lequel le noyau de renforcement (14) ou la pointe (16) du noyau de renforcement (14) est pré-courbé(e) dans la direction de la courbure du dispositif de restriction (1) en forme annulaire dans la position de fermeture, et le noyau de renforcement (14) comporte du métal ou du plastique dur.

9. Appareil selon l'une des revendications 1-8, dans lequel les échancrures (20) et l'ouverture (18) du dispositif de fermeture (8) sont adaptées mutuellement en complémentarité de formes.

10. Appareil selon l'une des revendications 1-9, dans lequel le dispositif de restriction (1) est constitué d'un matériau transparent ou coloré.

11. Appareil selon l'une des revendications 1-10, dans lequel au moins l'élément allongé (2) du dispositif de restriction (1) comporte une courbure prédéfinie par trempe à une température prédéfinie.
